# EUROPEAN PATENT SPECIFICATION

(11) **EP 2 629 713 B1**
(45) Date of publication and mention of the grant of the patent: **27.12.2017**
(21) Application number: 11773974.8
(22) Date of filing: 18.10.2011
(51) Int. Cl.: A61F 5/00

(54) **STOMACH-SPANNING GASTRIC IMPLANTS**
MAGENUMSPANNENDE GASTRISCHE IMPLANTATE
IMPLANTS GASTRIQUES S'ÉTIRANT DANS L'ESTOMAC

(30) Priority: 11.05.2011 US 201161485009 P; 19.10.2010 US 394592 P
(43) Date of publication of application: 28.08.2013
(60) Divisional application: 17200792.4
(73) Proprietor: APOLLO ENDOSURGERY, INC., Austin, TX 78746 (US)
(72) Inventor: BABKES, Mitchell, H., Santa Clarita California 91350 (US); DOMINGUEZ, Zachary, P., Santa Barbarra California 93101 (US)
(74) Representative: Hoffmann Eitle
(86) International application number: PCT/US2011/056614
(87) International publication number: WO 2012/054414

(56) References cited:
- WO-A2-2005/097012
- WO-A2-2005/110280
- US-A1- 2009 312 597
- US-A1- 2010 049 224
- US-A1- 2010 168 782

## Description

### Field of the Invention

The present invention is directed to intragastric implants used for the treatment of obesity, and in particular to implants for placement in and spanning the stomach cavity.

### Background of the Invention

Over the last 50 years, obesity has been increasing at an alarming rate and is now recognized by leading government health authorities, such as the Centers for Disease Control (CDC) and National Institutes of Health (NIH), as a disease. In the United States alone, obesity affects more than 60 million individuals and is considered the second leading cause of preventable death. Worldwide, approximately 1.6 billion adults are overweight, and it is estimated that obesity affects at least 400 million adults.

Obesity is caused by a wide range of factors including genetics, metabolic disorders, physical and psychological issues, lifestyle, and poor nutrition. Millions of obese and overweight individuals first turn to diet, fitness and medication to lose weight; however, these efforts alone are often not enough to keep weight at a level that is optimal for good health. Surgery is another increasingly viable alternative for those with a Body Mass Index (BMI) of greater than 40. In fact, the number of bariatric surgeries in the United States was estimated to be about 400,000 in 2010.

Examples of surgical methods and devices used to treat obesity include the LAP-BAND® (Allergan Medical of Irvine, CA) gastric band and the LAP-BAND AP® (Allergan). However, surgery might not be an option for every obese individual; for certain patients, non-surgical therapies or minimal-surgery options are more effective or appropriate.

In the early 1980s, physicians began to experiment with the placement of intragastric balloons to reduce the size of the stomach reservoir, and consequently its capacity for food. Once deployed in the stomach, the balloon helps to trigger a sensation of fullness and a decreased feeling of hunger. These devices are designed to provide therapy for moderately obese individuals who need to shed pounds in preparation for surgery, or as part of a dietary or behavioral modification program. These balloons are typically cylindrical or pear-shaped, generally range in size from 200-500 ml or more, are made of an elastomer such as silicone, polyurethane, or latex, and are filled with air, an inert gas, water, or saline.

One such inflatable intragastric balloon is described in U.S. Pat. No. 5,084,061 and is commercially available as the BioEnterics Intragastric Balloon System ("BIB System," sold under the trademark ORBERA). The BIB System comprises a silicone elastomer intragastric balloon that is inserted into the stomach and filled with fluid. Conventionally, the balloons are placed in the stomach in an empty or deflated state and thereafter filled (fully or partially) with a suitable fluid. The balloon occupies space in the stomach, thereby leaving less room available for food and creating a feeling of satiety for the patient. Placement of the intragastric balloon is non-surgical, trans-oral, usually requiring no more than 20-30 minutes. The procedure is performed gastroscopically in an outpatient setting, typically using local anesthesia and sedation. Placement of such balloons is temporary, and such balloons are typically removed after about six months. Removing the balloon requires deflation by puncturing with a gastroscopic instrument, and either aspirating the contents of the balloon and removing it, or allowing the fluid to pass into the patient's stomach. Clinical results with these devices show that for many obese patients, the intragastric balloons significantly help to control appetite and accomplish weight loss.

Some attempted solutions for weight loss by placing devices in the stomach result in unintended consequences. For instance, some devices tend to cause food and liquid to back up in the stomach, leading to symptoms of gastroesophageal reflux disease (GERD), a condition in which the stomach contents (food or liquid) leak backwards from the stomach into the esophagus. Also, the stomach acclimates to some gastric implant devices, leading to an expansion of stomach volume and consequent reduction in the efficacy of the device.

Therefore, despite many advances in the design of intragastric obesity treatment implants, there remains a need for improved devices that can be implanted for longer periods than before or otherwise address certain drawbacks of intragastric balloons and other such implants.

US2009312597 (A1) discloses a gastric bypass sleeve that includes an esophageal portion comprising a flexible material that conforms to the action of esophageal sphincter and a pyloric portion comprising a flexible material that conforms to the action of pyloric sphincter. The bypass sleeve has:
a) esophageal bendable sections at esophageal sphincter that allow substantial closing of sphincter, thereby preventing reflux from stomach to esophagus; and
b) pyloric bendable sections at pyloric sphincter that allow substantial closing of sphincter, thereby preventing reflux from intestinal to stomach.

Additionally, the bypass sleeve includes one or more openings designed to allow exchange of digestive material, including digestive enzymes from gastric tissue and/or nutrients from within the contents of gastric bypass sleeve.
The bypass sleeve includes at least one restrictor, for example being configured to adjustably tighten around sleeve, thereby selectively changing the passage speed of nutrients and/or enzymes, through sleeve.

WO2005110280 (A2) discloses a section of a sleeve device that passes through the pylorus has enough wall flexibility or compliance to allow normal opening and closing of the pylorus to release and retain stomach contents and to allow drainage of stomach secretions around the outside of the sleeve. This can optionally be accomplished by the inclusion of pleats, channels or other structures to facilitate the collapse and sealing of the sleeve as well as passage of gastric secretions along the outside of the sleeve.

### Summary of the Invention

The present invention addresses the above-described problems by providing passive intragastric apparatuses as defined by independent claim 1 for inducing satiety and therefore treating obesity.

Each of the implants described herein is formed of materials that permit it to be compressed into a substantially linear transoral delivery configuration and that will resist degradation over a period of at least six months within the stomach.

In accordance with a first embodiment, a passive intragastric obesity treatment implant comprises an esophageal stent sized to anchor within the esophagus just above the esophageal sphincter. A tubular body has a length sufficient to extend between the esophageal sphincter and the pyloric sphincter upon implant in the stomach, the tubular body having perforations therein to permit ingress of stomach juices. A duodenal tube extends in series from the tubular body. Collapsible tubular connectors extend between the esophageal stent and the tubular body, and between the tubular body and the duodenal tube, the connectors each having longitudinal slits therein. Finally, a bulbous flange surrounds and connects to the distal end of the tubular body, the bulbous flange having a size that prevents passage through the pyloric sphincter. The implant may further include an enlargement surrounding the duodenal tube and sized to prevent passage through the pyloric sphincter. The duodenal tube and enlargement may extend only up to 5-10 cm in length. The implant desirably further includes perforations along the tubular body to allow ingress of digestive stomach juices. The bulbous flange is preferably molded with relatively thick walls to maintain its as-molded shape without inflation. In one embodiment, the entire implant is made of silicone.

A further understanding of the nature and advantages of the invention will become apparent by reference to the remaining portions of the specification and drawings.

### Brief Description of the Drawings

Features and advantages of the present invention will become appreciated as the same become better understood with reference to the specification, claims, and appended drawings wherein:
Figure 1 is a sectional view through a patient's stomach illustrating an implanted stomach-spanning intragastric obesity treatment implant connected to a duodenal sleeve;
Figure 1A is a perspective view of the implant of Figure 1;
Figure 1B is an enlarged sectional view through a portion of Figure 1;
Figure 1C is an enlarged sectional view through an alternative distal end configuration for the implant shown in Figure 1;
Figure 2 is a sectional view through a patient's stomach illustrating a further implanted stomach-spanning intragastric obesity treatment implant having positioning rings and shelves, and attached to a duodenal sleeve;
Figure 3 is a sectional view through a patient's stomach illustrating a still further implanted stomach-spanning intragastric obesity treatment implant having an esophageal flange and a pyloric stent; and
Figure 4 is a perspective view of the implant of Figure 3.

### Detailed Description of the Preferred Embodiments

The present invention is directed to a variety of different intragastric implants that passively treat obesity by taking up space within the stomach or contact areas in and around the stomach to induce feelings of satiety. Such passive devices do not autonomously change shape, but instead react within the stomach to induce satiety. The devices may reduce volume within the stomach, thus reducing the digestive capacity. Additionally, the devices may contact areas within the stomach, such as the cardia surrounding the esophageal sphincter, to stimulate satiety-inducing nerves. Also, a number of devices slow gastric emptying by blocking or otherwise impeding flow through the pyloric sphincter. Other devices delay digestion by providing a duodenal sleeve. A number of devices combine two or more of these satiety-inducing features. Methods of implant are disclosed including compressing the devices within a delivery tube and transorally advancing the devices through the esophagus to be deployed within the stomach. Removal of the devices occurs in the reverse. Furthermore, some implants described herein affect the rate of stomach emptying. It should be understood that a number of the disclosed implants provide more than one of these passive aspects, and also that any disclosed structure could be combined with another disclosed structure unless physically impossible. As such, combinations of the passive satiety-inducing features disclosed herein, even if not explicitly stated, are contemplated. The term "passive" refers primarily to a lack of any moving parts within the implants, but in general to the inert nature of the various devices. A passive implant as defined herein, however, is not one that cannot affect change or stimulate the stomach, but rather one that may do so without any physical or chemical changes to its basic makeup.

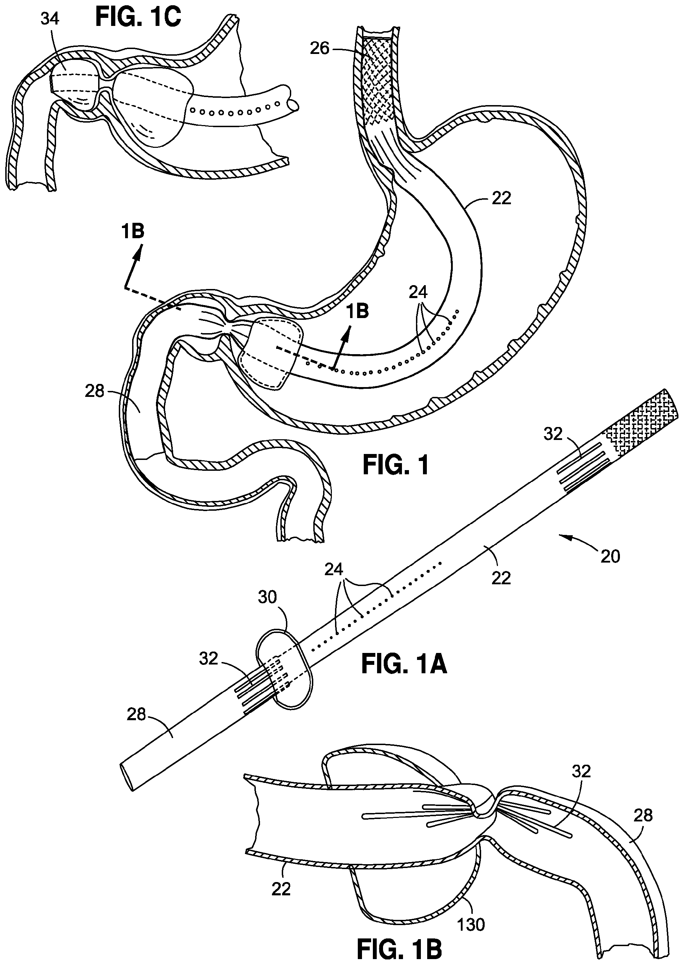

Figure 1 illustrates a first stomach-spanning implant 20, but also illustrates the anatomy of the human stomach, which will be described first. The major function of the stomach is to temporarily store food and release it slowly into the duodenum. The esophagus extending downward from the mouth connects to the stomach via esophageal sphincter, which regulates flow food into the stomach cavity. The cardia surrounds the superior opening of the stomach. The rounded portion superior to the body and adjacent the cardia is the fundus. Inferior to the fundus is the large central portion of the stomach, called the body, that is lined with muscles that contract and relax repetitively to churn the food therein. The stomach processes the food to a semi-solid "chyme," which enables better contact with the mucous membrane of the intestines, thereby facilitating absorption of nutrients. In addition, the stomach is an important site of enzyme production.

Lower down in the stomach the antrum connects the body to the pylorus, which leads into the duodenum. Below the stomach, the duodenum leads into the upper part of the small intestine (not shown); the jejunum makes up about one-third of the small intestine. The region of the stomach that connects to the duodenum is the pylorus. The pylorus communicates with the duodenum of the small intestine via the pyloric sphincter (valve). This valve regulates the passage of chyme from stomach to duodenum and it prevents backflow of chyme from duodenum to stomach.

One general category of passive satiety-inducing implants disclosed herein includes both a space-occupying member and a flow-through channels within the stomach through which solid and liquid flows. One way to look at such artificial intragastric spaces is that they create a stomach-within-stomach.

For example, Figures 1-1C disclose a implant 20 configured as a perforated tube 22 that is held in position within the biological stomach, bypassing it and acting as a small, artificial stomach, thereby decreasing the amount of food that is ingestible. Perforations 24 allow ingress of digestive stomach juices. An esophageal stent 26 is built into the tube 22 to anchor the proximal end within the esophagus. A distal sleeve 28 of the tube 22 extends past the pyloric sphincter and empties directly into the duodenum. Pyloric anchoring is achieved by the short duodenal sleeve 28, which also reduces nutrient absorption within the duodenum. To prevent the implant from migrating further down the duodenum, a bulbous flange 30 attaches to the tube 22 close to the distal sleeve 28. The bulbous flange 30 is too large to pass through the pyloric sphincter. Desirably, the bulbous flange 30 is molded with relatively thick walls to maintain its as-molded shape without inflation, and may includes holes to allow stomach juices to freely flow in and out.

At the locations where the tube 22 passes through the esophageal and pyloric sphincters, longitudinal slits 32 formed in the walls serve to allow compression of the sphincters and complete closure/sealing. That is, the slits 32 permit the tube 22 to easily buckle inward. Functionality of the slits 32 without damage to the sphincters is dependent on the conformity/pliability and softness of the material from which the tube 22 is manufactured. Desirably, the tube 22 is made of a resilient material that springs outward in the absence of sphincter closing forces, and thus the slits are in substantially constant contact with the surrounding anatomical walls which helps prevent leakage through the slits 32.

Some food will normally "leak" from the esophagus around the bypassing tube 22 into the biological stomach, through the slits, and/or around the esophageal stent 26. The leaked food will likely be small particulate and liquid only, as larger boluses will be naturally steered to and trapped within the tube 22.

Insertion and removal of the implant 20 is accomplished by inserting into the esophagus, a thin-walled, lubricated Teflon tube that is pre-loaded with the compressed implant 20. A distal end of the insertion tube is positioned using visualization techniques within the duodenum, at which point the implant 20 is held from linear movement while the insertion tube is retracted. The esophageal stent 26 anchors and locates the implant 20, and a small amount of repositioning prior to deploying the stent may be required as a final step of implanting the implant 20. To remove, the stent 20 will be grabbed and constricted inward, whereupon the rest of the implant 20 may be withdrawn without too much difficulty.

Figure 1C is an enlarged sectional view through an alternative distal end configuration for the implant shown in Figure 1. In this embodiment, the duodenal sleeve 28 is replaced with a much shorter tube enclosed within an enlargement 34, such as an inflated or pillow-like structure. The enlargement 34 prevents migration of the distal to back into the stomach. Although not shown, the short flow-through tube may be extended as far as the sleeve 28 shown in Figure 1 to reduce the ability of the duodenum to absorb nutrients, thus slowing digestion. In one embodiment, the short duodenal tube and enlargement 34 extend only up to 5-10 cm into the duodenum.

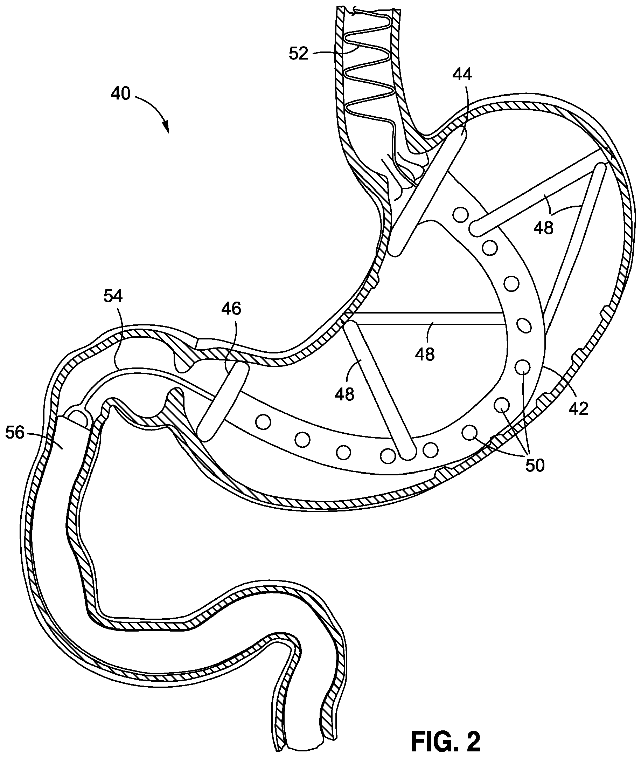

Another so-called stomach-in-stomach implant 40 seen in Figure 2 provides weight control in three ways - by stimulating the cardia, by providing a stomach-in-stomach, and by providing a duodenal sleeve. The implant 40 comprises an elongated tube 42 having a length that permits it to extend in a curve as show generally from the esophageal sphincter to the pylorus. A proximal shelf 44 surrounding an open proximal end of the tube 42 stabilizes the implant at the esophageal sphincter, while a distal shelf 46 surrounding a distal opening accomplishes the same thing adjacent at the pylorus. These shelves 44, 46 prevent migration back up the esophagus and down the pylorus, respectively. Furthermore, the upper shelf 44 rests firmly against the cardia walls, applying pressure thereto and thereby triggering release of satiety-inducing hormones, signaling the body to stop eating.

The tube 42 is highly flexible and includes a plurality of positioning rings 48 attached thereto, preferably molded into the side wall of the tube. The positioning rings 48 are also highly flexible, so the entire structure can be compressed down into a lubricated introduction tube. The positioning rings provide struts that help maintain the curvature of the tube 42 within the stomach, as shown - in other words, the tube 42 extends in a gradual arc from the esophageal sphincter to the pylorus rather than taking the shortest path. As such, there is preferably at least one positioning ring 48, and more preferably two positioning rings, on the inside curve of the tube 42 to maintain spacing from the lesser curvature of the stomach. Likewise, there is preferably at least one positioning ring 48, and more preferably two positioning rings, on the outside curve of the tube 42 to maintain spacing from the cardia region and greater curvature. Solids and liquids swallowed by the patient enter the tube 42 through the proximal shelf 44 and pass therethrough to exit through the distal shelf 46. Since the tube 42 can hold much less volume than the stomach, smaller than normal amounts of nutrients are able to be processed.

The tube 42 further includes a plurality of fluid transfer perforations 50 that allow digestive juices to freely flow in and out of the tube. Furthermore, peristaltic convolutions of the stomach apply mechanical forces through the tube 42 walls, desirably through the positioning rings 48, to help break down food boluses. Some leakage of food exiting the esophagus into the larger, biological stomach will likely occur, and leakage out through the pylorus will also likely occur. However, most ingested food will likely make its way through this channeling system.

An esophageal stent 52 connected to the proximal shelf 44 helps maintain the preferred position of the implant 40 within the stomach. The stent 52 may be balloon-or self-expanding, and in the illustrated embodiment comprises a helical coil of plastic wire. The esophageal sphincter is allowed to close as normally as possible, since the spiral plastic stent 52 is molded very thinly in the area that passes centrally through the sphincter.

The satiety-inducing implant 40 also restricts caloric intake through the duodenum, as the bottom segment is anchored via an intermediate tether 54 by a duodenal sleeve 56 that lines the upper duodenal wall. Such a duodenal sleeve 56 partially prevents nutrient absorption by inhibiting or delaying the point at which chyme from the stomach contacts the mucous membranes of the intestine.

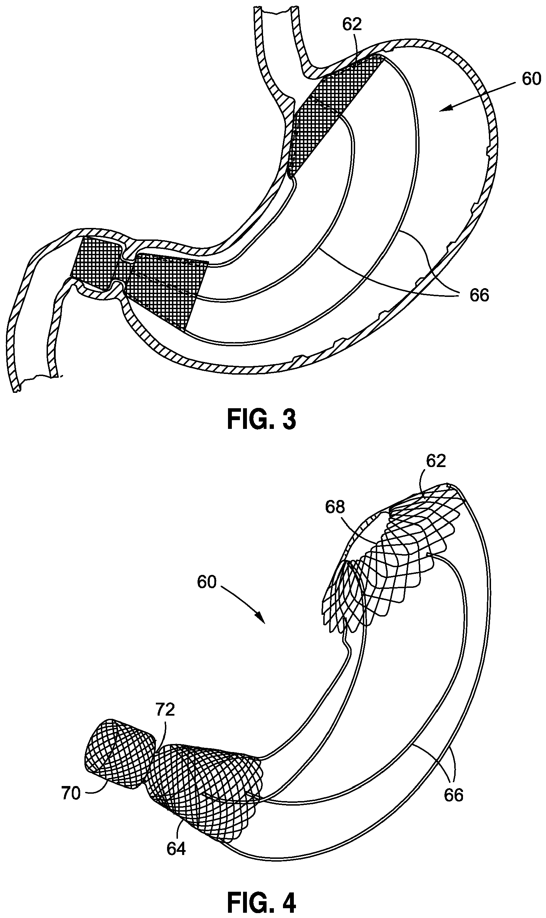

Figure 3 illustrating a different stomach-spanning intragastric obesity implant 60 having a cardia flange 62 and a generally tubular antrum stent 64 connected by struts 66, while Figure 4 shows the implant by itself. The cardia flange 62 is a flexible, flat partial conical ring that includes a central through hole 68 centered at the esophageal sphincter through which food passes. In the illustrated embodiment the cardia flange 62 comprises a braided wire mesh, such as from Nitinol covered with a soft silicone. Likewise, the antrum stent 64 comprises a braided wire mesh, such as from Nitinol covered with a soft silicone. It should be noted that other configurations for the cardia flange 62 and the antrum stent 64 are contemplated; for instance, they may alternatively be a solid silicone member with wire reinforcements.

In the illustrated example, the implant 60 further comprises a duodenal stent 70 connected in series with the antrum stent 64 at a narrow neck region 72. The combined somewhat hourglass shape of the antrum stent 64, neck region 72, and duodenal stent 70 is adapted to conform closely to the antrum, pylorus and upper end of the duodenum. The duodenal stent 70 is considered optional.

The struts 66 connecting the cardia flange 62 and antrum stent 64 are preferably flexible enough to straighten out and be passed transorally down an access tube, while also being somewhat stiff to provide light pressure to both the cardia flange 62 and antrum stent 64 on each end. In this way, the cardia flange 62 contacts and stimulates the cardia, and antrum stent 64 contacts and stimulates the antrum, both helping to induce a feeling of satiety. In a preferred embodiment there is no esophageal stent, and the entire device resides below the esophageal sphincter. As stated above, the duodenal stent 70 is optional and in a configuration without it the implant 60 remains anchored in place just by virtue of the stiffness of the struts 66 applying pressure to both the cardia flange 62 and antrum stent 64.

In one example, the central through hole 68 of the cardia flange 62 is large enough to avoid impeding flow of food and liquid into the stomach. Likewise, the antrum stent 64 and duodenal stent 70 may be sized to permit free flow of chime, or may be slightly undersized so as to delay gastric emptying, and thus slow the eating process. All of the tubular elements and the length of the struts 66 may be custom sized to fit a variety of patients.

As with the embodiments, the implant 60 is implanted transorally, across the gastroesophageal (G-E) junction, during a minimally invasive gastroendoscopic surgical procedure. The implant 60 may easily be compressed within a delivery tube and advanced through the esophagus to be deployed within the stomach. The Nitinol stents easily compress down to pass through the access tube, with the stuts 66 therebetween. One sequence includes first expelling the antrum stent 64, and duodenal stent 70 if included, at the antrum, and gradually withdrawing the access tube to release the struts 66 and then the cardia flange 62. Withdrawal through a similar tube using a grabber is also contemplated.

It should also be stated that any of the embodiments described herein may utilize materials that improve the efficacy of the implant. For example, a number of elastomeric materials may be used including, but not limited to, rubbers, fluorosilicones, fluoroelastomers, thermoplastic elastomers, or any combinations thereof. The materials are desirably selected so as to increase the durability of the implant and facilitate implantation of at least six months, and preferably more than 1 year.

Material selection may also improve the safety of the implant. Some of the materials suggested herein, for example, may allow for a thinner wall thickness and have a lower coefficient of friction than the implant.

The implantable devices described herein will be subjected to clinical testing in humans. The devices are intended to treat obesity, which is variously defined by different medical authorities. In general, the terms "overweight" and "obese" are labels for ranges of weight that are greater than what is generally considered healthy for a given height. The terms also identify ranges of weight that have been shown to increase the likelihood of certain diseases and other health problems. Applicants propose implanting the devices as described herein into a clinical survey group of obese patients in order to monitor weight loss.

The clinical studies will utilize the devices described above in conjunction with the following parameters.

### Materials:

a. Silicone materials used include 3206 silicone for any shells, inflatable structures, or otherwise flexible hollow structures. Any fill valves will be made from 4850 silicone with 6% BaSo₄. Tubular structures or other flexible conduits will be made from silicone rubber as defined by the Food and Drug Administration (FDA) in the Code of Federal Regulations (CFR) Title 21 Section 177.2600.

### Purposes:

i. the devices are for human implant,
ii. the devices are intended to occupy gastric space while also applying intermittent pressure to various and continually changing areas of the stomach;
iii. the devices are intended to stimulate feelings of satiety, thereby functioning as a treatment for obesity.

### General implant procedures:

i. The device is intended to be implanted transorally via endoscope into the corpus of the stomach.
ii. Implantation of the medical devices will occur via endoscopy.
iii. Nasal/Respiratory administration of oxygen and isoflurane to be used during surgical procedures to maintain anesthesia as necessary.

One exemplary implant procedure is listed below.
i. Perform preliminary endoscopy on the patient to examine the GI tract and determine if there are any anatomical anomalies which may affect the procedure and/or outcome of the study.
ii. Insert and introducer into the over-tube.
iii. Insert a gastroscope through the introducer inlet until the flexible portion of the gastroscope is fully exited the distal end of the introducer.
iv. Leading under endoscopic vision, gently navigate the gastroscope, followed by the introducer/over-tube, into the stomach.
v. Remove gastroscope and introducer while keeping the over-tube in place.
vi. *OPTIONAL:* Place the insufflation cap on the over-tubes inlet, insert the gastroscope, and navigate back to the stomach cavity.
vii. *OPTIONAL:* Insufflate the stomach with air/inert gas to provide greater endoscopic visual working volume.
viii. Collapse the gastric implant and insert the lubricated implant into the over-tube, with inflation catheter following if required.
ix. Under endoscopic vision, push the gastric implant down the over-tube with gastroscope until visual confirmation of deployment of the device into the stomach can be determined.
x. Remove the guide-wire from the inflation catheter is used.
xi. If inflated: Inflate the implant using a standard BioEnterics Intragastric Balloon System ("BIB System") Fill kit.
xii. Using 50-60cc increments, inflate the volume to the desired fill volume.
xiii. Remove the inflation catheter via over-tube.
xiv. Inspect the gastric implant under endoscopic vision for valve leakage, and any other potential anomalies. Record all observations.
xv. Remove the gastroscope from over-tube.
xvi. Remove the over-tube from the patient.

### End Point Criteria:

- Weight Loss
- Comprehensive Metabolic Panel (CMP)
- HbA1C
- Lipid Panel
- Tissue Samples/Response

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention(especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, references may have been made to patents and printed publications in this specification. Each of the above-cited references and printed publications are individually incorporated herein by reference in their entirety.

Specific embodiments disclosed herein may be further limited in the claims using "consisting of" or "consisting essentially of" language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. A passive intragastric obesity treatment implant (20), comprising:
an esophageal stent (26) sized to anchor within the esophagus just above the esophageal sphincter;
a tubular body (22) having a length sufficient to extend between the esophageal sphincter and the pyloric sphincter upon implant in the stomach, the tubular body having perforations (24) therein to permit ingress of stomach juices;
a duodenal tube (28) extending in series from the tubular body;
collapsible tubular connectors between the esophageal stent and the tubular body, and between the tubular body and the duodenal tube, the connectors each having longitudinal slits (32) therein; and
a bulbous flange (30) surrounding and connected to a distal end of the tubular body, the bulbous flange having a size that prevents passage through the pyloric sphincter,
the implant being formed of materials that permit it to be compressed into a substantially linear transoral delivery configuration and that will resist degradation over a period of at least six months within the stomach.

2. The implant of claim 1, further including:
an enlargement surrounding the duodenal tube and sized to prevent passage through the pyloric sphincter.

3. The implant of claim 2, wherein the duodenal tube and enlargement extend only up to 5-10 cm in length.

4. The implant of claim 1, further including perforations along the tubular body to allow ingress of digestive stomach juices.

5. The implant of claim 1, wherein the bulbous flange is molded with relatively thick walls to maintain its as-molded shape without inflation.

6. The implant of claim 1, wherein the entire implant is made of silicone.

## Patentansprüche

1. Passives intragastrisches Implantat zur Obesitas-Behandlung (20), umfassend:
einen Ösophagusstent (26), bemessen zum Verankern in dem Ösophagus unmittelbar oberhalb des Ösophagussphinkters;
einen röhrenförmigen Körper (22), der eine ausreichende Länge aufweist, um sich zwischen dem Ösophagussphinkter und dem Pylorussphinkter nach Implantieren im Magen zu erstrecken, wobei der röhrenförmige Körper Perforationen (24) darin aufweist, um das Eindringen von Magensäften zuzulassen;
eine Duodenalsonde (28), die sich in Reihe von dem röhrenförmigen Körper erstreckt;
zusammenlegbare röhrenförmige Verbinder zwischen dem Ösophagusstent und dem röhrenförmigen Körper, und zwischen dem röhrenförmigen Körper und der Duodenalsonde, wobei die Verbinder jeweils Längsschlitze (32) darin aufweisen; und
einen bauchigen Flansch (30) umgebend und verbunden mit einem distalen Ende des röhrenförmigen Körpers, wobei der bauchigen Flansch eine Größe aufweist, die einen Durchgang durch den Pylorussphinkter verhindert,
wobei das Implantat aus Materialien gebildet ist, die zulassen, dass es zu einer im Wesentlichen linearen transoralen Abgabekonfiguration gedrückt wird und dass es Abbau über einen Zeitraum von mindestens sechs Monaten im Magen widerstehen wird.

2. Implantat nach Anspruch 1, weiterhin beinhaltend:
eine Vergrößerung, die die Duodenalsonde umgibt und bemessen ist, um einen Durchgang durch den Pylorussphinkter zu verhindern.

3. Implantat nach Anspruch 2, wobei die Duodenalsonde und Vergrößerung sich nur bis zu 5-10 cm lang erstrecken.

4. Implantat nach Anspruch 1, das weiterhin Perforationen entlang des röhrenförmigen Körpers enthält, um das Eindringen von verdauenden Magensäften zuzulassen.

5. Implantat nach Anspruch 1, wobei der bauchige Flansch mit relativ dicken Wänden geformt ist, um seine ursprünglich geformte Form ohne Inflation beizubehalten.

6. Implantat nach Anspruch 1, wobei das gesamte Implantat aus Silikon besteht.

## Revendications

1. Implant intragastrique passif pour le traitement de l'obésité (20), comprenant :
un stent oesophagien (26) dimensionné pour s'ancrer dans l'oesophage juste au-dessus du sphincter oesophagien ;
un corps tubulaire (22) ayant une longueur suffisante pour s'étendre entre le sphincter oesophagien et le sphincter pylorique lors de l'implantation dans l'estomac, le corps tubulaire ayant des perforations (24) pour permettre l'entrée de sucs gastriques ;
un tubage duodénal (28) s'étendant en série à partir du corps tubulaire ;
des connecteurs tubulaires pliables entre le stent oesophagien et le corps tubulaire, et entre le corps tubulaire et le tubage duodénal, les connecteurs ayant chacun des fentes longitudinales (32) dans ceux-ci ; et
une bride bulbeuse (30) circonférentielle et reliée à une extrémité distale du corps tubulaire, la bride bulbeuse ayant une taille qui empêche tout passage à travers le sphincter pylorique,
l'implant étant formé de matériaux qui lui permettent d'être comprimé dans une configuration de distribution trans-orale substantiellement linéaire et qui résistera à une dégradation sur une période d'au moins six mois dans l'estomac.

2. Implant selon la revendication 1, incluant en outre :
un élargissement entourant le tubage duodénal et dimensionné pour empêcher tout passage à travers le sphincter pylorique.

3. Implant selon la revendication 2, dans lequel le tubage duodénal et l'élargissement ne s'étendent que jusqu'à 5 à 10 cm de longueur.

4. Implant selon la revendication 1, incluant en outre des perforations le long du corps tubulaire pour permettre l'entrée de sucs gastriques digestifs.

5. Implant selon la revendication 1, dans lequel la bride bulbeuse est moulée avec des parois relativement épaisses pour conserver sa forme moulée sans gonflement.

6. Implant selon la revendication 1, dans lequel l'implant entier est constitué de silicone.
